(19) Europäisches Patentamt
European Patent Office
Office européen des brevets

(11) **EP 2 692 331 A1**

(12) **EUROPEAN PATENT APPLICATION**
published in accordance with Art. 153(4) EPC

(43) Date of publication:
05.02.2014 Bulletin 2014/06

(21) Application number: 12765530.6

(22) Date of filing: 23.03.2012

(51) Int Cl.:
A61K 8/31 (2006.01)  A23L 1/30 (2006.01)
A23L 1/302 (2006.01)  A23L 1/303 (2006.01)
A61K 8/55 (2006.01)  A61K 8/60 (2006.01)
A61K 8/67 (2006.01)  A61K 9/107 (2006.01)
A61K 47/10 (2006.01)  A61K 47/12 (2006.01)
A61K 47/14 (2006.01)  A61Q 5/00 (2006.01)
A61Q 11/00 (2006.01)  A61Q 13/00 (2006.01)
A61Q 15/00 (2006.01)  A61Q 19/00 (2006.01)

(86) International application number:
PCT/JP2012/057633

(87) International publication number:
WO 2012/133246 (04.10.2012 Gazette 2012/40)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 30.03.2011 JP 2011074846

(71) Applicant: FUJIFILM Corporation
Tokyo 106-8620 (JP)

(72) Inventor: SERIZAWA, Shinichiro
Ashigarakami-gun
Kanagawa 258-8577 (JP)

(74) Representative: Morpeth, Fraser Forrest
Fujifilm Imaging Colorants Limited
Intellectual Property Group
P.O. Box 42
Hexagon Tower
Blackley, Manchester M9 8ZS (GB)

(54) **CAROTENOID-CONTAINING COMPOSITION AND METHOD FOR PRODUCING SAME**

(57) Provided are a carotenoid-containing composition containing at least one carotenoid; at least one selected from the group consisting of ascorbic acid, derivatives thereof and salts of the acid and the derivatives; and an emulsifier, the at least one selected from the group consisting of ascorbic acid, derivatives thereof and salts of the acid and the derivatives being contained in an amount, in terms of a number of moles of ascorbic acid, in a range of from 30 times to 190 times a total number of moles of the at least one carotenoid, and the composition having a pH in the range of from 6.5 to 9.0; and a method for preparing the composition.

EP 2 692 331 A1

**Description**

Technical Field

The invention relates to a carotenoid-containing composition and a method for producing the same.

Background Art

In recent years, attention has been paid to the high functionality of carotenoids, and various compositions containing carotenoids have been suggested. Since carotenoids are in general widely known as a sparingly soluble material, forms of emulsified composition are usually employed.

As an emulsified composition containing a carotenoid, for example, Japanese Patent Application Laid-Open (JP-A) No. 9-157159 discloses a carotenoid-containing composition in which, in order to increase the absorbability of carotenoids, an oil phase formed by melting carotenoids in oils and fats is emulsified in an aqueous phase containing a polyhydric alcohol in the presence of a polyglycerin fatty acid ester and lecithin, and the average particle size of the oil phase is 100 nm or less.

On the other hand, Japanese Patent No. 3846054 discloses a carotenoid-based coloring material-containing material containing a soluble eggshell membrane and a water-soluble anti-oxidant, for the purpose of sufficiently preventing discoloration of a carotenoid-based coloring material. Furthermore, Japanese Patent Application National Publication (Laid-Open) No. 2006-502127 discloses a technology of using solid particles of an ascorbic acid salt in a dispersant in which ascorbic acid is insoluble, as an antioxidizing agent for an oxidation-sensitive substance selected from carotenoids, retinoids, and unsaturated fatty acids.

[Prior Art Documents]

[Patent Documents]

Patent Document 1: JP-A No. 9-157159
Patent Document 2: Japanese Patent No. 3846054
Patent Document 3: Japanese Patent Application National Publication (Laid-Open) No. 2006-502127

SUMMARY OF INVENTION

Technical Problem

In regard to a carotenoid-containing composition, a further enhancement of storage stability is desired. The problem of a decrease in storage stability is conspicuous when the carotenoid-containing composition is an emulsified composition containing fine emulsion particles, or a powder composition. Among carotenoids, lycopene and fucoxanthin are unstable and are susceptible to oxidative decomposition, and therefore, a decrease in storage stability in carotenoid-containing compositions containing such a carotenoid poses a particularly serious problem.

However, it is a current situation that a carotenoid-containing composition which solves such a problem of storage stability has not been provided.

Therefore, it is an object of the invention to provide a carotenoid-containing composition having excellent storage stability, and a method for preparing the composition.

Solution to Problem

The invention relates to the following.

[1] A carotenoid-containing composition containing at least one carotenoid; at least one selected from the group consisting of ascorbic acid, derivatives thereof and salts of the acid and the derivatives; and an emulsifier, the at least one selected from the group consisting of ascorbic acid, derivatives thereof and salts of the acid and the derivatives being contained in an amount, in terms of a number of moles of ascorbic acid, in a range of from 30 times to 190 times a total number of moles of the at least one carotenoid, and the composition having a pH in the range of from 6.5 to 9.0.

[2] The carotenoid-containing composition as described in item [1], wherein the at least one carotenoid are at least one selected from lycopene or fucoxanthin.

[3] The carotenoid-containing composition as described in item [1] or [2], wherein the at least one selected from ascorbic acid, derivatives thereof and salts of the acid and the derivatives, is at least one selected from L-ascorbic acid, sodium L-ascorbate, or calcium L-ascorbate.

[4] The carotenoid-containing composition as described in any one of items [1] to [3], further comprising at least one selected from the group consisting of tocopherols and aromatic carboxylic acids, cinnamic acids and ellagic acids as phenolic antioxidizing agents.

[5] The carotenoid-containing composition as described in any one of items [1] to [4], further containing a water-soluble entrapping agent.

[6] The carotenoid-containing composition as described in any one of items [1] to [5], further containing a (poly) glycerin fatty acid ester having 1 to 6 glycerin units, 1 to 6 fatty acid units, and at least one hydroxyl group of a glycerin unit.

[7] The carotenoid-containing composition as described in any one of items [1] to [6], wherein the composition is an oil-in-water emulsion composition containing dispersed particles which have an average particle size in a range of from 50 nm to 300 nm and contain the at least one carotenoid.

[8] The carotenoid-containing composition as described in item [7], wherein the dispersed particles in the oil-in-water emulsion composition further contain the at least one selected from the group consisting of ascorbic acid, derivatives thereof and salts of the acid and the derivatives.

[9] The carotenoid-containing composition as described in any one of items [1] to [6], which is a powdered composition.

[10] A method for preparing the carotenoid-containing composition according to any one of items [1] to [7], the method including mixing an oil phase composition containing at least one carotenoid with an aqueous phase composition containing an emulsifier and at least one selected from the group consisting of ascorbic acid, derivatives thereof and salts of the acid and the derivatives; and emulsifying the mixture under pressure, to obtain an oil-in-water emulsion composition.

[11] The method as described in item [10], wherein the oil phase composition is obtained by mixing the at least one carotenoid with a (poly)glycerin fatty acid ester which has 1 to 6 glycerin units, 1 to 6 fatty acid units, and at least one hydroxyl group of a glycerin unit, to obtain an oil phase component mixed liquid; and then heating the oil phase component mixed liquid under temperature conditions of higher than or equal to the melting point of the at least one carotenoid.

[12] The method for preparing a carotenoid-containing composition as described in item [10] or [11], the method including drying the oil-in-water emulsion composition to obtain a powdered composition.

Advantageous Effects of Invention

According to the invention, a carotenoid-containing composition having excellent storage stability and a method for preparing the same can be provided.

DESCTIPTION OF EMBODIMENTS

Hereinafter, the carotenoid-containing composition of the invention and the method for preparing the composition will be described in detail.

The numerical value range described by using the expression "A to B" in the present specification describes a range including the numerical values A and B described before and after "to" as the minimum value and the maximum value, respectively.

In the invention, in the case of mentioning the amounts of various components in the composition, when plural substances corresponding to each of the various components in the composition are present, unless particularly stated otherwise, the amount means the total amounts of the relevant plural substances present in the composition.

The expression "(poly)glycerin fatty acid ester" according to the present specification includes all of a glycerin fatty acid ester containing one each of a glycerin unit and a fatty acid unit, a glycerin fatty acid ester containing a plural number of any one of a glycerin unit and a fatty acid unit, and a glycerin fatty acid ester containing a plural number of a glycerin unit and a plural number of a fatty acid unit. Thus, the expression is used in the case of using these glycerin fatty acid esters without distinction.

[Carotenoid-containing composition]

The carotenoid-containing composition of the invention contains at least one carotenoid; at least one selected from the group consisting of ascorbic acid, derivatives thereof and salts of the acid and the derivatives; and an emulsifier, the at least one selected from the group consisting of ascorbic acid, derivatives thereof and salts of the acid and the derivatives being contained in an amount, in terms of a number of moles of ascorbic acid, in a range of from 30 times to 190 times a total number of moles of the at least one carotenoid, and the composition having a pH in the range of from 6.5 to 9.0.

The carotenoid-containing composition of the invention exhibits excellent storage stability by having the constitution described above.

The carotenoid-containing composition of the invention may be in any form. Suitable examples of the form of the carotenoid-containing composition of the invention include an oil-in-water type emulsion composition, and a powdered composition obtained by drying the oil-in-water type emulsion composition. Particularly, the carotenoid-containing composition of the invention exhibits excellent storage stability even in the case of employing the form of a powdered composition.

Hereinafter, the various constituent elements in the carotenoid-containing composition of the invention will be described in detail.

<Carotenoid>

The carotenoid-containing composition of the invention contains a carotenoid.

A carotenoid according to the invention is a terpenoid-based coloring matter of yellow to red colors, and examples thereof include compounds derived from plants, algae, and bacteria. Furthermore, the carotenoids are not limited to compound from naturally occurring sources, and any compound obtainable according to a conventional method may be used.

Specific examples of the carotenoid according to the invention include lycopene, α-carotene, β-carotene, γ-carotene, δ-carotene, actinioerythrol, bixin, canthaxanthin, capsorubin, β-8'-apocarotenal (apocarotenal), β-12'-apocarotenal, xanthophylls (for example, astaxanthin, fucoxanthin, lutein, zeaxanthin, capsanthin, β-cryptoxanthin, and violaxanthin), fucoxanthin, and hydroxyl or carboxyl derivatives thereof. These may be used singly, or two or more kinds may be used in combination.

Suitable carotenoids include lycopene and fucoxanthin.

Among them, from the viewpoint that the relevant compound is known to have a high oxidation preventing effect, a high whitening effect and the like, and addition of the relevant compound to the raw materials of foods, cosmetics and pharmaceuticals, and to processed products thereof and the like has been hitherto desired, investigated and implemented, lycopene is preferred as a carotenoid.

Lycopene (optionally, may be referred to as "lycopene") is a carotenoid having a chemical formula: $C_{40}H_{56}$ (molecular weight 536.87) and belongs to the carotene family, which is one kind of carotenoid. Lycopene is a red coloring matter exhibiting the maximum absorption at 474 nm (acetone).

Lycopene has cis- and trans-isomers of conjugated double bonds at the center of the molecule, and examples include an all-transform, a 9-cis form, and a 13-cis form. However, in the invention, any of these may be used.

Lycopene may be contained in the carotenoid-containing composition of the invention in the form of a lycopene-containing oil or a lycopene-containing paste, both of which have been separated and extracted from natural products containing lycopene.

In natural products, lycopene is contained in tomatoes, persimmons, watermelons, and pink grapefruits, and the lycopene-containing oil described above may be an oil separated and extracted from these natural products.

Furthermore, lycopene according to the invention may be an extract from a natural product, or a product obtained by appropriately purifying this extract as necessary, or may be a synthetic product.

In the invention, lycopene extracted from tomatoes is particularly preferred from the viewpoints of product quality and productivity.

Furthermore, in the invention, tomato extracts that are widely commercially available can be used as the lycopene-containing oil or paste, and examples thereof include Lyc-O-Mato 15% and Lyc-O-Mato 6% sold by Sunbright Co., Ltd.; and LYCOPENE 18 sold by Kyowa Hakko Kogyo Co., Ltd.

A carotenoid according to the invention may be a simple substance, or may constitute a carotenoid together with an oil component (oil) used at the time of being extracted from a natural product.

A carotenoid according to the invention may be a crystalline carotenoid, or may be a non-crystalline carotenoid. When a carotenoid is a crystalline carotenoid, it is preferable that at least 90% by mass thereof be present in a non-crystalline state in the carotenoid-containing composition.

When a carotenoid is a crystalline carotenoid, since the crystalline carotenoid is present in a non-crystalline state in the composition, the effect that could be impaired if crystalline bodies are present is not impaired, and the absorbability of carotenoid components in the body can be increased.

Whether a crystalline carotenoid is in a non-crystalline state may be checked by using a known means for detecting the crystal structure. Furthermore, whether a carotenoid is a crystalline carotenoid may be checked by a conventional method, and for example, differential scanning calorimetry (DSC), observation with a polarized microscope, X-ray diffraction and the like can be utilized. A state in which detection of crystalline bodies cannot be confirmed by these known technologies can be defined as a non-crystalline state. Particularly, in the invention, it is preferable to check whether a carotenoid is in a non-crystalline state, based on the presence of a DSC endothermic peak. Specifically, when the temperatures for heat absorption and heat emission are determined by subjecting a substance to one cycle of temperature increase-temperature decrease (15°C/min) over a temperature range of 30°C to 200°C using DSC Q2000 (TA Instruments Japan, Inc.), after freeze-drying and removal of water in the case where the carotenoid-containing composition is an emulsion, or in a powdered state in the case where the carotenoid-containing composition is a powdered composition, and if the presence of an endothermic peak which is recognizable is not recognized, the substance is considered to be in a non-crystalline state.

Furthermore, when a carotenoid is a crystalline carotenoid, it is desirable if at least 90% to 100% by mass of the crystalline carotenoid is in a non-crystalline state, and in view of dynamic absorbability, it is preferable that 95% to 100% by mass of the crystalline carotenoid be in a non-crystalline state. For example, it can be confirmed that at least 90% by mass of crystalline carotenoid included in the carotenoids is in a non-crystalline state, by comparing the amount of heat absorption of an endothermic peak originating from carotenoid crystals in the carotenoid-containing composition of the invention measured by differential scanning calorimetry (DSC) with the amount of heat absorption of an endothermic peak of a standard carotenoid crystal.

Here, the term "crystalline carotenoid" does not indicate a specific carotenoid, but means a carotenoid compound which can exist as a crystalline body at any one temperature in the temperature range of -5°C to 35°C due to various factors such as the preparation method, treatment and storage of the crystalline body, in the case where the carotenoid compound is in the form of an oil or paste containing carotenoid. Particularly, among the carotenoids described above, lycopene, β-carotene, δ-carotene, zeaxanthin, lutein, astaxanthin, fucoxanthin and the like are carotenoids in which crystalline bodies are likely to exist.

Regarding carotenoids according to the invention, only a single compound may be used, or two or more kinds may be used in combination.

The content of carotenoids is preferably 0.1 % by mass to 5% by mass, more preferably 0.2% by mass to 4% by mass, and even more preferably 0.3% by mass to 3% by mass, relative to the total mass of solid components (all the components excluding water) in the carotenoid-containing composition. When the content of carotenoids is in this range, it can be further expected to have the effects provided by the carotenoids.

<Ascorbic acid, derivatives thereof, and salts of acid and derivatives>

The carotenoid-containing composition of the invention contains at least one selected from the group consisting of ascorbic acid, derivatives thereof, and salts of the acid and the derivatives.

Ascorbic acid may be in any of L-form, D-form and DL-form, but from the viewpoint of availability and the like, the L-form is preferred.

Examples of ascorbic acid salts, ascorbic acid derivatives and salts thereof include sodium L-ascorbate, potassium L-ascorbate, calcium L-ascorbate, an L-ascorbic acid phosphoric acid ester, magnesium salt of an L-ascorbic acid phosphoric acid ester, an L-ascorbic acid sulfuric acid ester, an L-ascorbic acid sulfuric acid ester disodium salt, an L-ascorbic acid stearic acid ester, L-ascorbic acid 2-glycoside, an L-ascorbic acid palmitic acid ester, and L-ascorbyl tetraisopalmitate; and fatty acid esters of ascorbic acid such as stearic acid L-ascorbyl ester, tetraisopalmitic acid L-ascorbyl ester, and palmitic acid L-ascorbyl ester.

Among the ascorbic acid, derivatives thereof, and salts of the acid and the derivatives described above, from the viewpoint of stability of a carotenoid, L-ascorbic acid, sodium L-ascorbate, calcium L-ascorbate, an L-ascorbic acid stearic acid ester, L-ascorbic acid 2-glucoside, an L-ascorbic acid palmitic acid ester, magnesium salt of an L-ascorbic acid phosphoric acid ester, an L-ascorbic acid sulfuric acid ester disodium salt, and L-ascorbyl tetraisopalmitate are preferred.

One particularly suitable embodiment of ascorbic acid, derivatives thereof, and salts of the acid and the derivatives is at least one selected from L-ascorbic acid, sodium L-ascorbate, and calcium L-ascorbate. These can be suitably incorporated into the carotenoid-containing composition even in the case of applying the carotenoid-containing composition of the invention to foods and the like.

In the carotenoid-containing composition of the invention, it is necessary that ascorbic acid and derivatives thereof be incorporated in an amount, in terms of a number of moles of ascorbic acid, in a range of from 30 times to 190 times a total number of moles of carotenoid, and it is preferable that ascorbic acid and derivatives thereof be incorporated in an amount of from 35 times to 150 times a total number of moles, and more preferably in an amount of from 40 times to 120 times a total number of moles.

Here, the amount in terms of a number of moles of ascorbic acid means that in regard to ascorbic acid among the ascorbic acid and derivatives thereof contained in a carotenoid-containing composition, the molar amount of ascorbic acid itself is employed, and in regard to an ascorbic acid derivative, a molar amount obtained by calculating the molar amount of the partial structure derived from ascorbic acid contained in the derivative relative to an equal amount of ascorbic acid.

The amount of ascorbic acid contained in the carotenoid-containing composition, and the amount of the partial structure derived from ascorbic acid in an ascorbic acid derivative can be quantitatively determined by designating a molecular weight corresponding to one skeletal structure of ascorbic acid as one mole.

For example, in the case of calcium ascorbate, since the calcium ascorbate is formed from two skeletal structures of ascorbic acid, the molecular weight of calcium ascorbate 390.34 is regarded as 2 moles, and 195.17 g is designated as 1 mole.

<Emulsifier>

The carotenoid-containing composition of the invention contains an emulsifier.

The emulsifier may be any of an anionic surfactant, a cationic surfactant, an amphoteric surfactant, and a nonionic surfactant.

Furthermore, from the viewpoint of the emulsifying power, the emulsifier preferably has an HLB value of 10 or higher, and more preferably 12 or higher. If the HLB value is too low, the emulsifying power may be insufficient. Meanwhile, from the viewpoint of a foam suppressing effect, an emulsifier having an HLB value of higher than or equal to 5 and lower than 10 may also be used in combination.

Here, the HLB is the hydrophilic-hydrophobic balance that is conventionally used in the field of surfactants, and a calculation formula that is conventionally used, for example, Kawakami's formula, can be used. Kawakami's formula is presented below.

$$HLB = 7 + 11.7 \log(M_w/M_0)$$

wherein $M_w$ represents the molecular weight of the hydrophilic group, and $M_0$ represents the molecular weight of the hydrophobic group.

Furthermore, the values of HLB described in catalogues and the like may also be used.

Also, as can be seen from the formula described above, an emulsifier having an arbitrary HLB value can be obtained by utilizing the additivity of the HLB.

The content of the emulsifier in the carotenoid-containing composition can be set depending on the form of the composition.

The content of the emulsifier in the carotenoid-containing composition is, in the case of employing the form of an emulsion composition, preferably 0.5% to 30% by mass, more preferably 1% to 20% by mass, and even more preferably 2% to 15% by mass, relative to the total amount of the composition, and in the case of employing the form of a powdered composition, the content is preferably 0.1% to 50% by mass, more preferably 5% to 45% by mass, and even more preferably 10% to 30% by mass, relative to the total amount of the composition. When the content is in this range, the interface tension between the oil phase the poor solvent phase is likely to decrease, and it is preferable from the viewpoint that using an excess amount can be avoided, and a problem of foaming of the emulsion composition becoming worse does not easily occur.

Furthermore, regarding the total mass of the emulsifier, when any form of the carotenoid-containing composition is to be employed, the emulsifier can be used in an amount in the range of 0.1 times to 10 times a total mass of oil components including a carotenoid. From the viewpoint of micronization of the dispersed particles and suppression of foaming in the case of preparing the carotenoid-containing composition into an emulsion composition, a total mass is preferably 0.5 times to 8 times, and particularly preferably 0.8 times to 5 times. When a total mass is in this range, the dispersion stability in the case of preparing the carotenoid-containing composition into an emulsion composition can be made satisfactory.

Among the emulsifiers, in view of being less irritant and having less adverse effects on the environment, a nonionic surfactant is preferred. Examples of the nonionic surfactant include a sucrose fatty acid ester, a polyglycerin fatty acid ester, an organic acid monoglyceride, a propylene glycol fatty acid ester, a polyglycerin condensed ricinoleic acid ester, a sorbitan fatty acid ester, and a polyoxyethylene sorbitan fatty acid ester.

Regarding the sucrose fatty acid ester, from the viewpoint of the stability of the dispersed particles in the case of preparing the carotenoid-containing composition into an emulsion composition, the number of carbon atoms in the fatty acid that constitutes the sucrose fatty acid ester is preferably 12 to 20, and more preferably 14 to 16.

Preferred examples of the sucrose fatty acid ester include sucrose dioleic acid ester, sucrose distearic acid ester, sucrose dipalmitic acid ester, sucrose dimyristic acid ester, sucrose dilauric acid ester, sucrose monooleic acid ester, sucrose monostearic acid ester, sucrose monopalmitic acid ester, sucrose monomyristic acid ester, and sucrose monolauric acid ester. Among these, sucrose monooleic acid ester, sucrose monostearic acid ester, sucrose monopalmitic acid ester, sucrose monomyristic acid ester, and sucrose monolauric acid ester are more preferred.

In the invention, these sucrose fatty acid esters can be used singly or as mixtures.

The carotenoid-containing composition of the invention can contain a polyglycerin fatty acid ester having an HLB value of 10 or higher as an emulsifier, apart from the particular polyglycerin fatty acid ester that will be described below.

Such a polyglycerin fatty acid ester is an ester between a polyglycerin having an average degree of polymerization of 2 or higher, preferably 6 to 15, and more preferably 8 to 10, and a fatty acid having 8 to 18 carbon atoms, for example, caprylic acid, capric acid, lauric acid, myristic acid, palmitic acid, stearic acid, oleic acid, and rinolic acid.

Preferred examples of the polyglycerin fatty acid ester include hexaglycerin monooleic acid ester, hexaglycerin monostearic acid ester, hexaglycerin monopalmitic acid ester, hexaglycerin monomyristic acid ester, hexaglycerin monolauric acid ester, decaglycerin monooleic acid ester, decaglycerin monostearic acid ester, decaglycerin monopalmitic acid ester, decaglycerin monomyristic acid ester, and decaglycerin monolauric acid ester.

Among these, more preferred examples include decaglycerin monooleic acid ester (HLB = 12), decaglycerin monostearic acid ester (HLB = 12), decaglycerin monopalmitic acid ester (HLB = 13), decaglycerin monomyristic acid ester (HLB = 14), and decaglycerin monolauric acid ester (HLB = 16).

These polyglycerin fatty acid esters can be used singly or as mixtures.

The sorbitan fatty acid ester according to the invention is preferably such that the number of carbon atoms of the fatty acid is 8 or greater, and more preferably 12 or greater. Preferred examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, and sorbitan trioleate.

In the invention, these sorbitan fatty acid esters can be used singly or as mixtures.

The polyoxyethylene sorbitan fatty acid ester is preferably such that the number of carbon atoms of the fatty acid is 8 or greater, and more preferably 12 or greater. Furthermore, the length of ethylene oxide (number of added moles) of the polyoxyethylene is preferably 2 to 100, and more preferably 4 to 50.

Preferred examples of the polyoxyethylene sorbitan fatty acid ester include polyoxyethylene sorbitan monocaprylate, polyoxyethylene sorbitan monolaurate, polyoxyethylene sorbitan monostearate, polyoxyethylene sorbitan sesquistearate, polyoxyethylene sorbitan tristearate, polyoxyethylene sorbitan isostearate, polyoxyethylene sorbitan sesquiisostearate, polyoxyethylene sorbitan oleate, polyoxyethylene sorbitan sesquioleate, and polyoxyethylene sorbitan trioleate.

These polyoxyethylene sorbitan fatty acid esters can be used singly or as mixtures.

Furthermore, a phospholipid such as lecithin may also be incorporated as the emulsifier according to the invention.

The phospholipid that can be used in the invention is a compound having a glycerin skeletal structure, and fatty acid residues and phosphoric acid residues as essential constituent components, and having a base, a polyhydric alcohol or the like bonded thereto. The phospholipid is also referred to as lecithin. Since phospholipids have hydrophilic groups and hydrophobic groups in the molecule, phospholipids have been hitherto widely used as emulsifiers in the fields of foods, pharmaceuticals, and cosmetics.

Industrially, products having a lecithin purity of 60% or higher are utilized as lecithin, and those products can also be utilized in the invention. However, from the viewpoints of the formation of fine oil droplet particle size and the stability of functional oil components, the lecithin is preferably a product that is generally referred to as high purity lecithin, and this has a lecithin purity of 80% or higher, and more preferably 90% or higher.

Regarding the phospholipids, various conventionally known phospholipids that have been extracted and separated from the living bodies of plants, animals and microorganisms may be used.

Specific examples of such phospholipids include, for example, various lecithins derived from plants such as soybean, maze, peanut, rapeseed, and barley; animals such as egg yolk and cattle; and microorganisms such as Escherichia coli.

Examples of such lecithins indicated in their compound names include glycerolecithins such as phosphatidic acid, phosphatidylglycerin, phosphatidylinositol, phosphatidylethanolamine, phosphatidylmethylethanolamine, phosphatidylcholine, phosphatidylserine, bisphosphatidic acid, and diphosphatidylglycerin (cardiolipin); and sphingolecithins such as sphingomyelin.

Furthermore, in the invention, in addition to the high purity lecithins described above, hydrogenated lecithins, enzymatically decomposed lecithins, enzymatically decomposed hydrogenated lecithins, hydroxylecithins and the like can be used. These lecithins that can be used in the invention can be used singly or in the form of a mixture of plural kinds.

<pH>

The pH of the carotenoid-containing composition of the invention is in the range of from 6.5 to 9.0, preferably from 6.5 to 8.0, and more preferably from 7.0 to 8.0.

The adjustment of the pH of the carotenoid-containing composition can be carried out by addition of a compound which exhibits alkalinity by addition, decomposition or reaction with an alkali agent, and addition of an alkali agent is more preferred.

Examples of the alkali agent include sodium hydroxide, potassium hydroxide, calcium hydroxide, sodium carbonate, potassium carbonate, magnesium carbonate, ammonium carbonate, sodium hydrogen carbonate, and ammonium hydrogen carbonate. These alkali agents may be used singly, or two or more kinds thereof may also be used in combination. From the viewpoint of storage stability, sodium carbonate is more preferred as the alkali agent.

The amount of addition of the alkali agent can be appropriately set according to the pH of the carotenoid-containing composition.

When the carotenoid-containing composition of the invention is a liquid material, the pH is a pH value measured for the liquid material at 25°C.

Meanwhile, when the carotenoid-containing composition is a powdered composition, when the pH value measured at 25°C using an aqueous solution of the powdered composition at a solid concentration of 17.7% by mass is in the range of from 6.5 to 9.0, the powdered composition is included in the carotenoid-containing composition of the invention. However, when the powdered composition does not dissolve to a solid concentration of 17.7% by mass, if the pH of a saturated aqueous solution measured at 25°C is in the range of from 6.5 to 9.0, the powdered composition is included in the carotenoid-containing composition of the invention.

<Phenolic antioxidizing agent and tocopherol compound>

The carotenoid-containing composition of the invention preferably contains at least one selected from the group consisting of tocopherols and aromatic carboxylic acids, cinnamic acids and ellagic acids as phenolic antioxidizing agents (hereinafter, appropriately referred to as a "particular antioxidizing agent").

The phenolic antioxidizing agent and the tocopherol compound, which are the particular antioxidizing agents, may be used singly, or two or more kinds may be used in combination. In the case of using two or more kinds of the particular antioxidizing agents, two or more kinds selected from any one class of the phenolic antioxidizing agent and the tocopherol compound may be used, or two or more kinds selected from both classes may also be used.

«Phenolic antioxidizing agent»

The phenolic antioxidizing agent according to the invention is at least one selected from the group consisting of aromatic carboxylic acids, cinnamic acids, and ellagic acids.

Such a phenolic antioxidizing agent has one phenolic hydroxyl group in the molecule, and in the case where a crystalline carotenoid is applied as a carotenoid, the phenolic antioxidizing agent suppresses decomposition or loss of the crystalline carotenoid at the time of the heating treatment for melting the crystals of the crystalline carotenoid, and thereby enables utilization of the carotenoid with high efficiency.

Examples of the aromatic carboxylic acids include gallic acid (3,4,5-hydroxybenzoic acid) and derivatives thereof. Examples of the derivatives of gallic acid (3,4,5-hydroxybenzoic acid) include gallic acid esters such as propyl gallate, epicatechin gallate, and epigallocatechin gallate; and gallic acid glycosides such as gallotannin.

Examples of the cinnamic acids include ferulic acid and chlorogenic acid, and derivatives thereof. Derivatives of ferulic acid and chlorogenic acid include ferulic acid esters. Specific examples include ferulic acid, $\gamma$-oryzanol (rice bran extract), caffeic acid (caffeic acid or 3,4-dihydroxycinnamic acid), chlorogenic acid, glyceryl ferulic acid, and dihydroferulic acid.

Examples of the ellagic acids include ellagic acid.

Furthermore, the phenolic antioxidizing agent is preferably a low molecular weight compound from the viewpoint of the stability of the carotenoid components, and for example, the molecular weight is preferably 100 to 3000, and more preferably 100 to 1000.

The phenolic antioxidizing agent is preferably a cinnamic acid compound from the viewpoint of the stability of the carotenoid components, and among others, ferulic acid or $\gamma$-oryzanol, which are all obtainable from a rice bran extract, and mixtures thereof are more preferred.

When a phenolic antioxidizing agent is incorporated as the particular antioxidizing agent, the total content thereof in the composition may be any amount effective for suppressing decomposition or loss of the carotenoid components, and the total content can be adjusted to 1.3 times to 15.0 times a molar amount of the carotenoid components. The total content is preferably adjusted to 2 times to 10 times, and more preferably 3 times to 8 times, a molar amount of the carotenoid components. When the total content of the phenolic antioxidizing agent is 1.3 times or more of a molar amount of the carotenoid components, the amount is sufficient for exhibiting an effect of suppressing decomposition or loss of the carotenoid components, and when the total content is 15.0 times or less of a molar amount of the carotenoid components, incorporation of a sufficient amount of the carotenoid components can be achieved without failing.

There are no particular limitations on the tocopherol compound as the particular antioxidizing agent, and examples include compounds selected from a compound group including tocopherol and derivatives thereof, and a compound group including tocotrienol and derivatives thereof. These may be used singly, or plural kinds may be used in combination. Furthermore, compounds selected respectively from the compound group including tocopherol and derivatives thereof and the compound group including tocotrienol and derivatives thereof may be used in combination.

The compound group including tocopherol and derivatives thereof include dl-α-tocopherol, dl-β-tocopherol, dl-γ-tocopherol, dl-δ-tocopherol, dl-α-tocopherol acetate, dl-α-tocopherol nicotinate, dl-α-tocopherol linolate, dl-α-tocopherol succinate, and the like. Among these, dl-α-tocopherol, dl-β-tocopherol, dl-γ-tocopherol, dl-δ-tocopherol, and mixtures thereof (mixed tocopherols) are more preferred. Also, as the tocopherol derivatives, carboxylic acid esters thereof, particularly acetic acid esters, are preferably used.

The compound group including tocotrienol and derivatives thereof include α-tocotrienol, β-tocotrienol, γ-tocotrienol, δ-tocotrienol, and the like. Furthermore, as the tocotrienol derivatives, acetic acid esters thereof are preferably used.

When a phenolic antioxidizing agent is incorporated as the particular antioxidizing agent, the total content thereof in the composition is preferably 0.1 % by mass to 10% by mass, more preferably 0.5% by mass to 8.0% by mass, and even more preferably 1.0% by mass to 5.0% by mass.

<Water-soluble entrapping agent>

When the carotenoid-containing composition of the invention is prepared into a powdered composition that is obtainable by drying an emulsion composition, in order to protect oil droplets during the process of powderization at the time of drying, or at the time of powder storage, it is preferable that the composition contain a water-soluble entrapping agent. Thereby, the oil droplet particle size can be maintained finely, and at the same time, deterioration of the carotenoid components in the oil droplets can be reduced.

Furthermore, the water-soluble entrapping agent can improve the water dispersibility of oil components when the powdered composition is redissolved in water, and also, can improve the transparency after re-dissolution.

Examples of the water-soluble entrapping agent include monosaccharides, disaccharides and polysaccharides, such as glucose, fructose, lactose, maltose, sucrose, dextrins, maltodextrin (including flour candy), cyclodextrin, maltose, fructose, and trehalose.

One suitable embodiment of the water-soluble entrapping agent may be trehalose.

Furthermore, another suitable embodiment of the water-soluble entrapping agent may be at least one polysaccharide selected from fructose polymers and oligomers, each including sugar units containing at least two fructose units (hereinafter, simply referred to as "fructose polymers or oligomers").

Trehalose is a non-reducing disaccharide in which two molecules of D-glucose are bonded through an α,α-1,1 linkage. In the invention, high-purity hydrated crystalline trehalose can be suitably used.

Trehalose can be produced by, for example, a method of culturing yeast in a glucose solution, causing the yeast cells to produce trehalose within the cells, and separating this trehalose from the yeast cells; the van't Hoff method of culturing a bacterium in a glucose solution, causing the bacterium to produce trehalose in the culture fluid, and separating this trehalose from the culture fluid; or the like.

Furthermore, commercially available products of trehalose may also be used, and the commercially available products can be purchased from, for example, Hayashibara Co., Ltd.

The fructose polymer or oligomer refers to a polymer or oligomer which includes fructose as a repeating unit, and is also composed of a sugar unit in which plural sugar units are bonded by dehydration condensation. In the invention, a compound having fewer than 20 repeating units of sugar including fructose units is referred to as a fructose oligomer, and a compound having 20 or more of repeating units of sugar including fructose units is referred to as a fructose polymer.

The number of repetition of this sugar unit is preferably 2 to 60, and more preferably 4 to 20, from the viewpoints of suitability to drying and oil droplet micronization at the time of re-dissolution. When the number of repetition (degree of polymerization of fructose) is 2 or greater, hygroscopicity does not become excessively strong, and a decrease in the collection ratio caused by adhesion of the polymer or oligomer to the drying vessel during the drying process can be effectively prevented. On the other hand, when the number of repetition is 60 or less, coarsening of the oil droplet particle size at the time of re-dissolution in water can be effectively prevented.

The fructose polymer or oligomer may contain, in addition to fructose, another monosaccharide at the ends of the molecule or within the chain. Examples of the other monosaccharide unit include glucose, galactose, mannose, idose, altrose, gulose, talose, allose, xylose, arabinose, lyxose, ribose, threose, erythrose, erythrulose, xylulose, ribulose, psicose, sorbose, and tagatose, but the monosaccharide unit is not intended to be limited to these. Among these monosaccharides, glucose is preferred from the viewpoint of easy availability. Furthermore, it is preferable that the bonding position be present at the ends of the fructose chain, from the viewpoint of oil droplet micronization at the time of re-dissolution.

When the fructose polymer or oligomer contains a saccharide other than fructose, the content percentage is, as the degree of polymerization, 50% or less, and preferably 30% or less, relative to the number of fructose units from the viewpoints of suitability to drying and oil droplet micronization at the time of re-dissolution.

From the viewpoints of the storage stability of the coloring matter and easy availability, examples of the water-soluble entrapping agent that is preferably used in the invention include trehalose and inulin, and it is also preferable to use these in combination.

Inulin according to the invention refers to a fructose polymer or a fructose oligomer, both having one glucose unit at an end. Inulin is known to exist widely in nature, and is contained in large amounts in chicory, Jerusalem artichoke, dahlia, garlic, leek, onion, and the like. The details of inulin are described in Handbook of Hydrocolloids, G.O. Phillips, P.A. Williams, Ed., 397-403 (2000) CRC Press. In general, the chain length of a glucose unit is expressed by G, and the chain length of a fructose unit by F. Inulin of the invention does not  include sucrose, which is expressed by GF.

Inulin that is usually extracted from natural products is a polymer or an oligomer of from GF2 (kestose), GF3 (nystose) and GF4 (fructosylnystose) to about GF60, or a mixture thereof.

In the invention, inulin may include products that are obtained by separating and extracting with hot water from the roots of chicory, Jerusalem artichoke, dahlia and the like, concentrating this aqueous solution, and powderizing the aqueous solution by spray drying, and marketed. Examples thereof include FRUITAFIT (manufactured by Imperial Sensus LLC) extracted from the roots of chicory, BENEO (Beneo Orafti SA) similarly extracted from the roots of chicory, reagents derived from Dahlia roots (Wako Pure Chemical Industries, Ltd., and Sigma-Aldrich Co.), and reagents extracted from chicory roots (Sigma-Aldrich Co.).

Furthermore, the fructose oligomer and polymer according to the invention may also include products prepared from sucrose by utilizing the fructan transfer activity of β-fructofuranosidase. Examples of these products include FUJI FF (manufactured by Fuji Nihon Seito Corp.) and GF2 (manufactured by Meiji Seika Kaisha, Ltd.).

Inulin that is used in the invention is preferably such that, from the viewpoint of micronization of oil droplets at the time of re-dissolving, the number of repetition of fructose (degree of polymerization) is 2 to 60. More preferably, from the viewpoints of the adherence to the apparatus at the time of spray drying and the solubility in water, the degree of polymerization of fructose is 4 to 20.

Furthermore, another example of the water-soluble entrapping agent may be another water-soluble polymer or oligomer. Examples of the other water-soluble polymer and oligomer include, but are not limited to, agarose, starch, carrageenan, gelatin, xanthan gum, gellan gum, galactomannan, casein, tragacanth gum, xyloglucan, β-glucan, curdlan, water-soluble soybean fiber, chitosan, alginic acid, and sodium alginate.

The water-soluble entrapping agent is preferably added at the time of emulsification when the emulsion composition is prepared, but it is also acceptable to add a portion thereof or the entirety after emulsification.

The content of the water-soluble entrapping agent in the carotenoid-containing composition is, from the viewpoints of shape retention and solubility, preferably 0.5 times to 50 times, more preferably 1 time to 20 times, even more preferably 1 time to 10 times, and still more preferably 2 times to 5 times, relative to the total mass of the oil components in the composition.

Meanwhile, it is desirable if the water-soluble entrapping agent is contained in the  aqueous phase of the emulsion composition which is a carotenoid-containing composition, and in regard to the method for preparing a carotenoid-containing composition of the invention that will be described below, the water-soluble entrapping agent may be included as an aqueous composition at the time of emulsification under pressure, or may be added to the aqueous phase of the carotenoid-containing composition after emulsification under pressure.

<Particular (poly)glycerin fatty acid ester>

The carotenoid-containing composition of the invention preferably contains a (poly)glycerin fatty acid ester having 1 to 6 glycerin units, 1 to 6 fatty acid units, and at least one hydroxyl group of a glycerin unit (hereinafter, appropriately referred to as a "particular (poly)glycerin fatty acid ester"). The particular (poly)glycerin fatty acid ester is particularly suitably incorporated in the case where a crystalline carotenoid is applied as a carotenoid.

Meanwhile, the "emulsifier" according to the invention does not include the particular (poly)glycerin fatty acid ester.

Such a particular (poly)glycerin fatty acid ester exhibits high compatibility with the crystalline carotenoid in the case where a crystalline carotenoid is applied as a carotenoid, and lowers the melting point of the crystalline carotenoid. Also, in a co-melt of the particular (poly)glycerin fatty acid ester and the crystalline carotenoid, recrystallization of the crystalline carotenoid is suppressed.

In a (poly)glycerin fatty acid ester having 7 or more glycerin units, hydrophilicity increases, and the affinity with a carotenoid is decreased. On the other hand, in a (poly)glycerin fatty acid ester having 7 or more fatty acid units, the effect of suppressing carotenoid crystals cannot be expected. Furthermore, in a (poly)glycerin fatty acid which does not contain a hydroxyl group of a glycerin unit, for example, a medium-chain fatty acid triglyceride or the like, although the reason is not clearly understood, crystallization of carotenoid cannot be sufficiently suppressed, a certain amount of hydroxyl groups is required, and the effect of suppressing carotenoid crystals cannot be expected.

The particular (poly)glycerin fatty acid ester is, from the viewpoint of suppression of recrystallization and the like, preferably an ester between glycerin having a number of glycerin units (average degree of polymerization) of 1 to 6, and more preferably 1 to 4, and a fatty acid having 1 to 6 fatty acid units, and more preferably 1 to 5 fatty acid units, as well as 8 to 22 carbon atoms (for example, caprylic acid, capric acid, lauric acid, myristic acid, palmitic acid, stearic acid, oleic acid, rinolic acid, and behenic acid), and more preferably 14 to 18 carbon atoms.

Among these particular (poly)glycerin fatty acid esters, from the viewpoint of uniform solubility at the time of co-melting, a compound having a molecular weight of 10,000 or less is preferred, a compound having a molecular weight of 3000 or less is more preferred, and a compound having a molecular weight of 2500 or less is even more preferred. Furthermore, from the viewpoint of the affinity with a carotenoid, a compound having an HLB value of 9 or less is preferred, and a compound having an HLB value of 6 or less is more preferred.

Furthermore, when the carotenoid-containing composition is prepared into a powdered composition obtainable by drying an emulsion composition, it is preferable that the particular (poly)glycerin fatty acid ester be a particular (poly) glycerin fatty acid ester which is solid at normal temperature, from the viewpoints of the carotenoid concentration in the powdered composition and the yield at the time of hot air drying during the preparation of the composition. That is, when the composition is solid at normal temperature, it is not necessary to increase the amount of the entrapping agent, and a sufficient amount of carotenoids can be incorporated. Furthermore, when the composition is solid at normal temperature, the composition does not easily adhere to the contact surface at the time of hot air drying, and a decrease in the yield of the powdered composition can be suppressed. Examples of such particular (poly)glycerin fatty acid esters which are solid at normal temperature include glyceryl myristate, glyceryl monostearate, glyceryl distearate, diglyceryl monostearate, tetraglyceryl monostearate, tetraglyceryl tristearate, tetraglyceryl pentastearate, hexaglyceryl monostearate, hexaglyceryl tristearate, hexaglyceryl tetrabehenate, and hexaglyceryl pentastearate, all of which do not have branches and unsaturated bonds at the carbon atoms of the fatty acids.

Examples of the particular (poly)glycerin fatty acid ester that can be applied to the carotenoid-containing composition of the invention include glyceryl myristate, monoglyceryl monostearate, diglyceryl monostearate, triglyceryl monostearate, hexaglyceryl pentastearate, triglyceryl dipalmitate, glyceryl distearate, tetraglyceryl tristearate, tetraglyceryl pentastearate, hexaglyceryl monostearate, hexaglyceryl tristearate, and hexaglyceryl tetrabehenate. From the viewpoints of suppression of recrystallization and uniform solubility, glyceryl myristate, glyceryl monostearate, diglyceryl monostearate, tetraglyceryl pentastearate, and hexaglyceryl pentastearate are preferred.

The content (mass) of the particular (poly)glycerin fatty acid ester may vary depending on the kind or content of the crystalline carotenoid used, but from the viewpoint of stability of the carotenoid-containing composition, the content is preferably 0.01 times to 10 times, more preferably 0.1 times to 8 times, and even more preferably 0.3 times to 5 times, a total mass of the crystalline carotenoid. When the total mass of the particular polyglycerin fatty acid ester in the carotenoid-containing composition is 0.01 times a total mass of the crystalline carotenoid, a sufficient crystal suppressing effect can be expected, and when the total mass is 10 times or less, an increase in the particle size of the dispersed particles when an emulsion is formed can be suppressed.

<Other components>

The carotenoid-containing composition of the invention may also contain, in addition to the various components described above, other oil components that are conventionally used as oil phase components.

These other oil components are not particularly limited as long as they are components that do not dissolve in aqueous media but dissolve in oily media, and any components having properties or functionality suitable for the purpose can be appropriately selected and used. For example, unsaturated fatty acids, oil-soluble vitamins such as oils and fats such as coconut oil, and ubiquinones are preferably used.

Examples of the unsaturated fatty acids include monovalent highly unsaturated fatty acids (ω-9, oleic acid and the like) or polyvalent highly unsaturated fatty acids (ω-3 and ω-6), both having 10 or more carbon atoms, and preferably 18 to 30 carbon atoms. These unsaturated fatty acids may be any of known unsaturated fatty acids, and examples of ω-3 oils and fats include linoleic acid, eicosapentaenoic acid (EPA), docosahexaenoic acid (DHA), and fish oil and the like containing these.

Examples of the ubiquinones include coenzyme Q's such as coenzyme Q10.

Examples of the oil-soluble vitamins include vitamin A's, vitamin D's, and oil-solubilized derivatives of erythorbic acid. Here, the oil-soluble vitamins that are contained as the other components do not include tocopherol that can be incorporated as the particular antioxidizing agent.

Examples of vitamin A's include retinol, 3-hydroretinol, retinal, 3-hydroretinal, retinoic acid, 3-dehydroretinoic acid, and vitamin A acetate. Examples of vitamin D's include vitamin D's such as vitamins $D_2$ to $D_7$. Furthermore, examples of other oil-soluble vitamin substances include esters such as vitamin E nicotinate; and vitamin K's such as vitamins $K_1$ to $K_3$.

Further examples of the oil-soluble vitamins include fatty acid esters of erythorbic acid, such as palmitic acid erythorbyl ester and tetraisopalmitic acid erythorbyl ester; and fatty acid esters of vitamin $B_6$, such as pyridoxine dipalmitate, pyridoxine tripalmitate, pyridoxin dilaurate, and pyridoxine dioctanoate.

Examples of oils and fats other than those described above include liquid oils and fats (fatty oils) and solid oils and fats (fats) at normal temperature.

Examples of the liquid oils and fats include olive oil, camellia oil, macademia nut oil, castor oil, avocado oil, evening primrose oil, turtle oil, corn oil, mink oil, rapeseed oil, egg yolk oil, sesame oil, persic oil, wheat germ oil, sasanqua oil, linseed oil, safflower oil, cotton seed oil, perilla oil, soybean oil, peanut oil, tea seed oil, Japanese nutmeg oil, rice bran oil, Chinese tung oil, Japanese tung oil, jojoba oil, germ oil, triglycerin, glycerin trioctanoate, glycerin triisopalmitate, salad oil, safflower oil, palm oil, coconut oil, peanut oil, almond oil, hazelnut oil, walnut oil, grape seed oil, squalene, and squalane.

Furthermore, examples of the solid oils and fats include beef tallow, hardened beef tallow, cow leg fat, beef bone fat, mink oil, egg yolk oil, lard, horse fat, sheep fat, hardened oil, cacao fat, coconut fat, hardened coconut fat, palm oil, hardened palm oil, Japanese wax, Japanese wax kernel oil, and hardened castor oil.

Among those described above, from the viewpoints of the particle size and stability of the emulsion composition, coconut oil, which is a medium-chain fatty acid triglyceride, is preferably used.

<Other additive components>

In addition to the components described above, components that are conventionally used in the fields of foods and the like may be appropriately incorporated to the carotenoid-containing composition of the invention, depending on the form of the relevant composition.

When the carotenoid-containing composition is an emulsion composition, the additive components may be incorporated as components for an oil phase component mixed liquid, a carotenoid-containing oil phase composition, or an aqueous composition depending on the characteristics of the additive components, or may also be incorporated as additive components to the aqueous phase of the carotenoid-containing composition.

Such other components include polyhydric alcohols such as glycerin and 1,3-butylene glycol; monosaccharides or polysaccharides such as glucose, fructose, lactose, maltose, sucrose, pectin, κ-carrageenan, locust bean gum, guar gum, hydroxypropyl guar gum, xanthan gum, gum karaya, tamarind seed polysaccharides, gum arabic, tragacanth gum, hyaluronic acid, sodium hyaluronate, sodium chondroitin sulfate, and dextrin; sugar alcohols such as sorbitol, mannitol, maltitol, lactose, maltotriitol, and xylytol; inorganic salts such as sodium chloride and sodium sulfate; proteins having molecular weights of greater than 5000, such as casein, albumin, methylated collagen, hydrolyzed collagen, water-soluble collagen, and gelatin; synthetic polymers such as carboxyvinyl polymers, sodium polyacrylate, polyvinyl alcohol, polyethylene glycol, and ethylene oxide-propylene oxide block copolymers; water-soluble cellulose derivatives such as hydroxyethyl cellulose and hydroxymethyl cellulose; flavonoids (catechin, anthocyanin, flavones, isoflavone, flavan, flavanone, and rutin), phenolic acids (chlorogenic acid, ellagic acid, gallic acid, and propyl gallate), lignans, curcumins, and coumarins. These components may be incorporated as, for example, functional components, excipients, viscosity adjusting agents, and radical scavengers, based on their functions.

In addition to that, for example, other additives that are usually used for the applications such as various efficacious components, pH buffering agents, ultraviolet absorbers, antiseptic agents, fragrances, and colorants can also be used in combination.

(Oil-in-water type emulsion composition and powdered composition)

As described above, the carotenoid-containing composition of the invention may be prepared into an oil-in-water type emulsion composition obtained by mixing an oil phase composition and an aqueous phase composition by emulsification, or may be prepared into a powdered composition obtained by drying the oil-in-water type emulsion composition.

«Oil phase composition»

The oil phase composition preferably contains at least one carotenoid, and also contains oil components selected from the various components that are included in the constituent components of a carotenoid-containing composition.

Ascorbic acid, derivatives thereof, and salts of the acid and the derivatives may be included as the constituent components of an oil phase composition.

Furthermore, the particular antioxidizing agent and the (poly)glycerin fatty acid ester are preferably included as the constituent components of the oil phase composition.

Examples of carotenoids include those compounds described as a component of the carotenoid-containing composition, and suitable examples thereof also include the same.

Examples of ascorbic acid, derivatives thereof, and salts of the acid and the derivatives include those compounds described as components of the carotenoid-containing composition, and suitable examples thereof also include the same.

Examples of the particular antioxidizing agent include those compounds described as a component of the carotenoid-containing composition, and suitable examples thereof also include the same.

The content of the oil phase composition is, in the case of an emulsion composition, preferably 0.1 % by mass to 50% by mass, more preferably 0.5% by mass to 25% by mass, and even more preferably 0.2% by mass to 10% by mass, from the viewpoint of exhibiting the functions of the oil components. Furthermore, in the case of a powdered composition, the content of the oil phase composition is preferably 10% by mass to 50% by mass, more preferably 10% by mass to 40% by mass, and even more preferably 10% by mass to 30% by mass, relative to the total mass of the powdered composition.

When the composition is prepared into an oil-in-water type emulsion composition, the composition may also contain an emulsifier that can be used as an oil phase component, in addition to the components described above. Examples of the emulsifier that can be used as such an oil phase component include emulsifiers having HLB values of 7 or less among the emulsifiers described above.

«Aqueous phase composition»

It is preferable that the aqueous phase composition be composed of an aqueous medium, particularly water, and at least contain at least one selected from ascorbic acid, derivatives thereof and salts of the acid and the derivatives, and an emulsifier.

It is preferable that the water-soluble entrapping agent and the alkali agent be incorporated into the aqueous phase composition.

Examples of ascorbic acid, derivatives thereof, and salts of the acid and the derivatives include those compounds described as components of the carotenoid-containing composition, and suitable examples thereof also include the same.

Examples of the emulsifier include those compounds described as a component of the carotenoid-containing composition, and suitable examples thereof also include the same.

The aqueous phase composition may contain a polyglycerin fatty acid ester having an HLB value of 10 or higher, apart from the particular polyglycerin fatty acid ester described above.

When the carotenoid-containing composition of the invention is an oil-in-water type emulsion composition, the average particle size of the dispersed particles contained in the emulsion composition is preferably in the range of from 50 nm to 300 nm, and from the viewpoint of transparency, the average particle size is more preferably in the range of from 50 nm to 200 nm, and even more preferably in the range of from 50 nm to 150 nm.

Furthermore, when the carotenoid-containing composition of the invention is a powdered composition obtained by drying the oil-in-water type emulsion composition, the average particle size of the dispersed particles contained in the oil-in-water type emulsion composition obtained by redissolving the powdered composition by adding an aqueous medium such as water thereto, is preferably in the range of from 50 nm to 300 nm, and from the viewpoint of transparency, the average particle size is more preferably in the range of from 50 nm to 200 nm, and even more preferably in the range of from 50 nm to 150 nm.

Here, the average particle size of the dispersed particles means the particle size of dispersed particles (oil droplets) in the emulsion composition, and in the case of a powdered composition, the average particle size means the particle size of dispersed particles obtained when the powdered composition is prepared into a 1 % by mass liquid (at the time of re-dissolution).

The particle size of the dispersed particles can be measured with a commercially available particle size distribution meter or the like. Regarding the particle size distribution analysis method for an emulsion, an optical microscopic method, a confocal laser microscopic method, an electron microscopic method, an atomic force microscopic method, a static light scattering method, a laser diffraction method, a dynamic light scattering method, a centrifugal sedimentation method, an electric pulse measurement method, a chromatographic method, an ultrasonic attenuation method and the like are known, and apparatuses coping with the respective principles are commercially available.

In view of the particle size range according to the invention and ease of measurement, a dynamic light scattering method is preferred for the particle size measurement of the dispersed particles in the invention. Examples of commercially available measuring apparatuses using dynamic light scattering include NanoTrack UPA (Nikkiso Co., Ltd.), dynamic light scattering type particle size analyzer LB-550 (Horiba, Ltd.), and concentrated system particle size analyzer FPAR-1000 (Otsuka Electronics Co., Ltd.); however, for the particle size according to the invention, a value measured at 25°C using a particle size analyzer, FPAR-1000 (Otsuka Electronics Co., Ltd.), is employed.

That is, in regard to the method for measuring the particle size, in the case of an oil-in-water type emulsion composition, pure water is added to 20 times the volume, while in the case of a powdered composition, pure water is added to a solid concentration of 1% by mass, the particle size is determined as the median diameter (d = 50), using a particle size analyzer, FPAR-1000 (Otsuka Electronics Co., Ltd.).

Furthermore, the particle size of the dispersed particles can be adjusted by, in addition to the components of the composition, factors such as the stirring conditions (shear force, temperature, and pressure) in the preparation method, and the ratio of the oil phase and the aqueous phase.

(Applications)

The carotenoid-containing composition of the invention is a carotenoid-containing composition which has excellent storage stability and is sufficiently expected to have the desired effects induced by a carotenoid. Therefore, the carotenoid-containing composition can be preferably applied to food compositions, cosmetic compositions, and pharmaceutical compositions.

In the foods or cosmetics containing the carotenoid-containing composition of the invention, components that can be added to foods or cosmetics can be appropriately added as necessary. When the carotenoid-containing composition is used particularly in foods, long-term storage as foods in a powder form is enabled, and when the carotenoid-containing composition is dissolved in an aqueous medium, a dispersion composition having fine dispersed particles and excellent transparency is obtained.

Foods, cosmetics and the like that contain the carotenoid-containing composition of the invention can exhibit an effect that may not be sufficiently exhibited due to the presence of crystal bodies, for example, satisfactory absorbability of a carotenoid.

Regarding the cosmetic compositions, the carotenoid composition is suitably used in, for example, skin lotions, essences, emulsions, cream pack masks, cosmetic packs, hair cleansing cosmetics, fragrance cosmetics, liquid body washes, UV care cosmetics, deodorant cosmetics, and oral care cosmetics.

Furthermore, regarding the foods, general food products such as nutritional drinks, analeptics, palatable beverages, and ice candies, as well as nutritional supplement foods in the form of tablets, granules and capsules are also suitably used.

When the carotenoid-containing composition is used as a functional food product, the amount of addition of the powdered composition according to the invention may vary depending on the kind or purpose of the product and cannot be collectively defined; however, the powdered composition can be used by adding in an amount in the range of from 0.01 % to 10% by mass, and preferably from 0.05% to 5% by mass. When the amount of addition is 0.01 % by mass or more, exhibition of the intended effects can be anticipated, and when the amount of addition is 10% by mass or less, appropriate effects can be efficiently exhibited in many cases.

The carotenoid-containing composition of the invention can be suitably prepared by the method for preparing a carotenoid-containing composition (preparation method of the invention) that will be described below in detail.

[Method for preparing carotenoid-containing composition]

The carotenoid-containing composition of the invention is preferably prepared by a preparation method including mixing an oil phase composition containing a carotenoid, with an aqueous phase composition containing an emulsifier and at least one selected from the group consisting of ascorbic acid, derivatives thereof, and salts of the acid and the derivatives, emulsifying the mixture under pressure, and thereby obtaining an oil-in-water type emulsion composition (hereinafter, appropriately referred to as "preparation method of the invention").

Meanwhile, the details of the various components used in the preparation method of the invention have been described in connection with the various components contained in the carotenoid-containing composition of the invention, and suitable examples thereof have been likewise described.

When a crystalline carotenoid is incorporated as a carotenoid, a preferred embodiment for obtaining the oil phase composition is an embodiment including mixing a carotenoid with a (poly)glycerin fatty acid ester having 1 to 6 glycerin units, 1 to 6 fatty acid units, and at least one hydroxyl group of a glycerin unit to obtain an oil phase component mixed liquid (referred to as an oil phase component mixing step); and heating the oil phase component mixed liquid under the temperature conditions of higher than or equal to the melting point of the crystalline carotenoid components (referred to as an oil phase component heating step).

Meanwhile, even in the case wherein the carotenoid-containing composition contains a crystalline carotenoid as a carotenoid, it is not essentially necessary to carry out the preparation of the oil phase composition according to the embodiment described above including an oil phase component mixing step and an oil phase component heating step, and the embodiment of preparation of the oil phase composition is appropriately selected according to the kind of carotenoids.

When the oil phase composition is prepared by an embodiment including an oil phase component mixing step and an oil phase component heating step, since the oil phase component mixed liquid is heated at a temperature higher than or equal to the melting point of the crystalline carotenoid component, a carotenoid-containing composition in which recrystallization of the crystalline carotenoid among the carotenoid components is suppressed, and a non-crystalline state is stably maintained, can be obtained.

In the oil phase component mixing step, the various oil phase components constituting the carotenoid-containing composition as an oil phase composition are mixed. There are no particular limitations on the mixing of the oil phase components. An oil phase component mixed liquid is obtained by the oil phase component mixing step.

In the oil phase component heating step, the oil phase component mixed liquid is heated under the temperature conditions of higher than or equal to the melting point of the carotenoid components. The temperature employed when the oil phase component mixed liquid is heated is necessarily a temperature higher than or equal to the melting point of the carotenoid components. If the temperature is lower than the melting point of the carotenoid component, crystalline carotenoid does not melt, and a large amount of crystalline bodies come to exist in the carotenoid-containing composition. In the oil phase component heating step, since the crystalline carotenoid is co-melted together with the (poly)glycerin fatty acid ester, crystalline bodies can be melted at a lower temperature.

The melting point of a carotenoid component means a temperature at which crystalline carotenoid among the carotenoid component melts. When the carotenoid-component is composed of a simple crystalline carotenoid, the melting point corresponds to the melting point of the crystalline carotenoid. On the other hand, when a component other than a crystalline carotenoid is included in the carotenoid component, the melting point means the temperature at which carotenoids in the carotenoid component melt.

For example, when a carotenoid-containing oil derived from a natural product is used as a carotenoid component, impurities and the like may be included therein, and it is known that a crystalline carotenoid among the carotenoid components melts at a temperature lower than the melting point of the crystalline carotenoid. In this case, the temperature at which the crystalline carotenoid among the carotenoid components melts corresponds to the "melting point of the carotenoid components" according to the invention.

The melting point of the carotenoid components can be checked by a method which is generally used to check the melting point, and for example, the melting point can be checked by DSC.

The heating temperature (co-melting temperature) that is applied to the oil phase component heating step may vary, specifically depending on the kind of the crystalline carotenoid or carotenoid component used and the like; however, in general, in the case of carotenoid components including lycopene, the heating temperature can be set to from 150°C to 200°C, and from the viewpoint of suppressing thermal decomposition, the heating temperature is preferably from 150°C to 180°C, and more preferably from 150°C to 170°C.

Furthermore, the maximum heating temperature that is applied to the oil phase component heating step is such that, from the viewpoint of suppressing decomposition of crystalline carotenoids, the difference between the maximum temperature in a heating treatment and the melting point of the carotenoid components is 10°C or less, and the melting point is more preferably, for example, a temperature of 5°C or less.

The heating time that is applied to the oil phase component heating step may be any time in which the carotenoid component in the oil phase component mixed liquid melts, and from the viewpoint of efficiently suppressing non-crystallization of crystalline bodies and decomposition of crystalline carotenoids caused by excessive heat, the heating time is preferably from 10 minutes to 60 minutes, and more preferably from 15 minutes to 45 minutes, but is not limited thereto.

Meanwhile, in the oil phase component heating step, since it is important to arrange the entire oil phase component mixed liquid be at a uniform temperature, it is preferable to sufficiently stir the mixed liquid while heating the mixed liquid, and it is preferable to heat the mixed liquid using a sealed container while stirring the mixed liquid and to thereby maintain the mixed liquid at a constant temperature.

Through the oil phase component heating step, an oil phase composition is obtained.

In the preparation method of the invention, an emulsion composition is obtained by mixing an oil phase composition containing a carotenoid, with an aqueous phase composition containing an emulsifier and at least one selected from the group consisting of ascorbic acid, derivatives thereof, and salts of the acid and the derivatives, and emulsifying the mixture under pressure (emulsifying step).

Thereby, an oil droplets-in-water type emulsion composition in which oil phase components including carotenoid components are finely dispersed as oil droplets (dispersed particles) in water, can be obtained. In this emulsion composition, the carotenoid components including crystalline carotenoids are maintained in a stable manner.

The ratio (mass) between the oil phase and the aqueous phase in emulsification is not particularly limited, but the ratio of oil phase/aqueous phase (% by mass) is preferably 0.1/99.9 to 50/50, more preferably 0.5/99.5 to 30/70, and even more preferably 1/99 to 20/80.

When the ratio of oil phase/aqueous phase is adjusted to 0.1/99.9 or higher, since the effective components are not reduced, there is a tendency that a problem in practical use does not occur with the emulsion composition, which is preferable. Also, when the ratio of oil phase/aqueous phase is adjusted to 50/50 or lower, a decrease in the emulsifier concentration does not occur, and there is a tendency that emulsification stability of the emulsion composition is not deteriorated, which is preferable.

Emulsification may be carried out by conducting a one-step emulsification process, but it is preferable to conduct an emulsification process of two or more steps from the viewpoint of obtaining uniform and fine dispersed particles.

Specifically, it is particularly preferable to use two or more kinds of emulsifying apparatuses in combination by a method of performing emulsification using a high pressure homogenizer or the like, in addition to a one-step emulsification process of achieving  emulsification using a conventional emulsifying apparatus which utilizes a shear action (for example, stirring with a stirrer or an impeller, a homomixer, or a continuous flow shear apparatus). When a high pressure homogenizer is used, the emulsion can be arranged to have more uniform microparticulate liquid droplets. Also, the emulsification process may be carried out several times for the purpose of obtaining liquid droplets of a more uniform particle size.

Regarding the emulsification technique that can be used herein, emulsification methods that are generally known, such as a natural emulsification method, an interfacial chemical emulsification method, an electrical emulsification method, a capillary emulsification method, a mechanical emulsification method, and an ultrasonic emulsification method, can all be used.

Methods that are known to be useful for micronizing dispersed particles include interfacial chemical emulsification methods such as a PIT emulsification method and a gel emulsification method. These methods have an advantage that less energy is consumed, and are adequate in the case of finely emulsifying a material which is susceptible to deterioration by heat.

Furthermore, as an emulsification method which is used for general purposes, a method using mechanical force, that is, a method of dividing oil droplets by applying a strong shear force from an external source, is applied. The most general apparatus that provides mechanical force is a high-speed, high-shear stirring machine. Regarding such a stirring machine, stirring machines called Homomixer, Disper Mixer and UltraMixer are commercially available.

Furthermore, as another mechanical emulsifying apparatus which is useful for micronization, a high pressure homogenizer is available, and various apparatuses are commercially available. Since a high pressure homogenizer can provide a large shear force as compared with stirring methods, micronization is enabled even if the amount of the emulsifier is relatively small.

High pressure homogenizers can be roughly classified into chamber type high pressure homogenizers having a fixed throttle unit, and homogeneous valve type high pressure homogenizers in which the extent of throttle opening is controlled.

Examples of the chamber type high pressure homogenizers include MICROFLUIDIZER (manufactured by Microfluidics Corp.), NANOMIZER (Manufactured by Yoshida Kikai Co., Ltd.), and ALTIMIZER (manufactured by Sugino Machine, Ltd.).

Examples of the homogenous valve high pressure homogenizers include a Gaulin  type homogenizer (manufactured by APV, Inc.), a Ranie type homogenizer (manufactured by Ranie Chemie GmbH), a high pressure homogenizer (manufactured by Niro Soavi S.p.A.), a homogenizer (manufactured by Sanwa Machine Co., Ltd.), a high pressure homogenizer (manufactured by Izumi Food Machinery Co., Ltd.), and an ultrahigh pressure homogenizer (manufactured by IKA Works GmbH).

As an emulsifying apparatus having a simple structure, which is a dispersing apparatus having a relatively high energy efficiency, an ultrasonic homogenizer is available. Examples of a high power output ultrasonic homogenizer capable of even manufacturing include Ultrasonic Homogenizer US-600, Ultrasonic Homogenizer US-1200T, Ultrasonic Homogenizer RUS-1200T, and Ultrasonic Homogenizer MUS-1200T (all manufactured by Nissei Corp.); and Ultrasonic Processor UIP2000, Ultrasonic Processor UIP-4000, Ultrasonic Processor UIP-8000, and Ultrasonic Processor UIP-16000 (all manufactured by Hielscher Ultrasonics GmbH). These high power output ultrasonic irradiation apparatuses are used at a frequency of 25 kHz or less, and preferably 15 kHz to 20 kHz.

Furthermore, as other known emulsification techniques, methods of using a static mixer, a microchannel, a micromixer, a membrane emulsifying apparatus and the like, which all do not have an external stirring unit and require only a small amount of energy, are also useful methods.

The temperature conditions at the time of performing emulsification dispersion in the invention are not particularly limited, but from the viewpoint of the stability of functional oil components, the temperature is preferably from 10°C to 100°C, and an appropriate preferred range can be selected based on the melting point and the like of the functional oil components handled.

Furthermore, in the case of using a high pressure homogenizer in the invention, it is preferable to perform the treatment at a pressure of preferably 50 MPa or higher, more preferably from 50 MPa to 280 MPa, and even more preferably from 100 MPa to 280 MPa.

Furthermore, regarding the emulsion liquid which is an emulsified dispersed composition, it is preferable to cool the emulsion liquid through a certain cooling machine within 30 seconds or less, and preferably 3 seconds or less, immediately after the passage through the chamber, from the viewpoint of retaining the particle size of the dispersed particles.

Furthermore, the preparation method of the invention may include drying the oil-in-water type emulsion composition obtained by the emulsification step and thereby obtaining a powdered composition (hereinafter, may be referred to as "powderization step"). This carotenoid-containing composition as a powdered composition is a composition in a powderized form with excellent storage stability. Furthermore, even in the case where the carotenoid-containing composition contains a crystalline carotenoid, the emulsion composition obtained by redissolving the powdered composition in an aqueous medium is a composition in which crystallization of the crystalline carotenoid is suppressed.

Regarding the drying techniques that are used in the powderization step, any known drying techniques can be used, and examples thereof include natural drying, drying by heating, hot air drying, high frequency drying, ultrasonic drying, drying under reduced pressure, vacuum drying, freeze-drying, and spray drying. These techniques may be used singly, but two or more techniques can also be used in combination.

In the invention, since many functional materials which are relatively vulnerable to heat are contained, drying under reduced pressure, vacuum drying, freeze-drying, and spray drying are preferred. Furthermore, a method of drying in a vacuum (under reduced pressure) while maintaining the temperature to be lower than or equal to 0°C and higher than or equal to the freezing temperature, which is another form of vacuum drying, is also preferable.

In the case of performing vacuum drying or drying under reduced pressure, it is preferable to perform drying, while concentration is repeated as the degree of vacuum is gradually increased, in order to avoid scattering due to bumping.

In regard to the preparation method of the invention, freeze-drying by which ice is sublimed from a material that is in a frozen state, and thereby moisture is eliminated, is preferred. In this freeze-drying method, usually, since the drying process proceeds at or below 0°C, and usually at about -20°C to -50°C, there is a major advantage that thermal degeneration of the material does not occur, and taste, color, nutrition, shape, texture and the like can be easily restored in the water restoration process, to the state before being dried.

Examples of commercially available freeze drying machines include, but are not limited to, Freeze-Dryer VD-800F (Taitec Corp.), FLEXI-DRY MP (FTS Systems, Inc.), DURATOP/DURASTOP (FTS Systems, Inc.), Takara Vacuum Freeze Dryer Model A (Takara ATM K.K.), Desk Freeze Dryer FD-1000 (Tokyo Rikakikai Co., Ltd.), Vacuum Freeze Dryer FD-550 (Tokyo Rikakikai Co., Ltd.), and Vacuum Freeze Dryer (Takara Seisakusho Co., Ltd.).

Furthermore, in regard to the preparation method of the invention, from the viewpoint of achieving a balance between production efficiency and product quality in the drying technique, a spray drying method is particularly preferred. Spray drying is one type of convection hot air drying. When a liquid composition is sprayed and dried as fine particles having a size of several hundred μm or less in hot air, and the fine particles drop inside a column, the liquid composition is collected as a solid powder. Although the material is temporarily exposed to hot air, since the time of exposure is very short, and since the heat is latent heat of evaporation of water, the temperature does not increase excessively, thermal degeneration of the material does not easily occur as in the case of freeze-drying, and the change caused by water restoration is also small. In the case of a material which is highly vulnerable to heat, cold air can also be supplied instead of hot air. In that case, although the drying capacity is lower, it is preferable from the viewpoint that drying can be realized in a milder manner.

Examples of commercially available spray dryers include Spray Dryer SD-1000 (Tokyo Rikakikai Co., Ltd.), Spray Dryer L-8i (Ohkawara Kakohki Co., Ltd.), Closed Spray Dryer CL-12 (Ohkawara Kakohki Co., Ltd.), Spray Dryer ADL310 (Yamato Scientific Co., Ltd.), Mini Spray Dryer B-290 (Buchi Corp.), PJ-MiniMax (Powdering Japan K.K.), and PHARMASD (Niro, Inc.).

Furthermore, for example, it is also preferable to produce granules having excellent handleability simultaneously with drying, using an apparatus capable of simultaneously carrying out drying and granulation, such as a fluidized bed granulation dryer MP-01 (Powrex Corp.) or a fluidized bed built-in spray dryer FSD (Niro, Inc.).

EXAMPLES

Hereinafter, the invention will be described by way of Examples, but the invention is not intended to be limited to these. Meanwhile, unless particularly stated otherwise, units indicated as "parts" and "percent (%)" in the following descriptions are on a mass basis.

<Preparation of oil phase composition>

The oil phase components described below were mixed and heated from room temperature to a temperature in the range of from 155°C to 160°C under stirring, and while the mixture was heated and retained for about 10 minutes at a temperature in the range of from 155°C to 160°C, the mixture was melted and then cooled. Thus, a carotenoid-containing oil phase composition 1 was obtained.

<Preparation of aqueous phase composition>

The aqueous phase components (except for sodium carbonate) described below were mixed and stirred to thereby dissolve, while being heated at 70°C. Subsequently, the mixture was subjected to coarse dispersing for 90 seconds with a 600-W ultrasonic homogenizer (US-150T manufactured by Nissei Corp.), and thus an aqueous composition 1 was obtained.

[Oil phase components 1]

| | |
|---|---|
| Lycopene paste (lycopene concentration: 18%) | 5.90 g |
| Diglyceryl monostearate | 0.59 g |
| 50% solution of calcium ascorbate | 4.72 g |
| Ferulic acid | 1.78 g |

[Aqueous phase components 1]

| | |
|---|---|
| Sucrose lauric acid ester | 11.10 g |
| Lecithin | 1.81 g |
| Trehalose | 6.55 g |
| Ascorbic acid | 7.97 g |
| Sodium ascorbate | 7.97 g |
| Sodium carbonate (added to emulsion liquid) | 7.07 g |
| Water | 246.90 g |

Meanwhile, LYCOPENE 18 (manufactured by Kyowa Wellness Co., Ltd.) was used as the lycopene paste; NIKKOL DGMS (HLB = 5.0, manufactured by Nikko Chemicals Co., Ltd.) was used as diglyceryl monostearate; RYOTO SUGAR ESTER L-1695 (HLB = 16, manufactured by Mitsubishi Kagaku Foods Corp.) was used as sucrose lauric acid ester; LECION P (manufactured by Riken Vitamin Co., Ltd.) was used as lecithin; a trehalose product manufactured by Hayashibara Co., Ltd. was used as trehalose; VISCORIN (STDM) manufactured by Daiichi Fine Chemical Co., Ltd. was used as ascorbic acid; sodium L-ascorbate manufactured by Daiichi Fine Chemical Co., Ltd. was used as sodium ascorbate; and a sodium carbonate product manufactured by Kanto Chemical Co., Inc. (food additive) was used as sodium carbonate.

The melting point of LYCOPENE 18 is 153°C (DSC endothermic peak value). The DSC endothermic peak value was measured using DSC Q2000 (TA Instruments Japan, Inc.).

<Preparation of emulsion>

The oil phase composition 1 was kept warm at 70°C while stirred, and the entire amount of the aqueous phase composition 1 that had been prepared as described above and kept warm at 70°C was added thereto. The mixture was dispersed for 3 minutes with a 600-W ultrasonic homogenizer, and thus a coarsely dispersed emulsion 1 was obtained (carotenoid concentration 0.354%).

Subsequently, the coarsely dispersed emulsion 1 was subjected to a high pressure emulsification treatment at a pressure of 245 MPa and at 30°C repeatedly for 4 times, using STARBURST MINI (manufactured by Sugino Machine, Ltd.), and thus an emulsion liquid 1a was obtained.

Furthermore, sodium carbonate was added under stirring to the emulsion liquid 1a thus obtained to be dissolved therein, and thus an emulsion 1, which was a carotenoid-containing oil phase composition, was obtained (carotenoid concentration 0.36%).

Subsequently, the emulsion 1 thus obtained was spray dried using spray drying (Spray Dryer ADL310 type, manufactured by Yamato Scientific Co., Ltd.) under the conditions of a spray pressure of 0.15 MPa, an exit temperature of 80°C, and a throughput of 7 ml/min, and then a powder was collected with a cyclone. Thus, a powdered composition 1, which was a carotenoid-containing oil phase composition with a carotenoid concentration of 2.00%, was obtained.

The carotenoid-containing oil phase composition 1, the emulsion 1, and an aqueous solution of the powdered composition 1, which were obtained as described above, were subjected to an observation with a polarized microscope, and the effect of a carotenoid source on the presence of crystals was evaluated.

The observation with a polarized microscope was carried out using PCLIPSE LV100POL (manufactured by Nikon Corp.), for the carotenoid-containing oil phase composition 1 and the emulsion 1, by visually observing the materials themselves, and for the powdered composition 1, by dispersing the powdered composition in water to a solid concentration of 1% to form an emulsion and visually observing the emulsion. As a result, it was confirmed that the lycopene source, which was a crystalline carotenoid, was mostly not recognized. This implies that at least 90% by mass or more of the crystalline carotenoid was in a non-crystalline state.

[Example 2]

<Preparation of oil phase composition>

Among the oil phase components described below, mixed tocopherol and ferulic acid were added to glycerin tri(caprylate/caproate), and the mixture was dissolved and dispersed while heated in a constant temperature water bath at 70°C, and thus a dispersion liquid was obtained. Subsequently, a fucoxanthin paste was added to the dispersion liquid thus obtained, and the mixture was sufficiently stirred and dissolved. Thus, a carotenoid-containing oil phase composition 2 was obtained.

<Preparation of aqueous phase composition>

The aqueous phase components (except for sodium carbonate) described below were mixed and stirred to dissolve while heated at 70°C, and then the mixture was roughly  dispersed for 90 seconds with a 600-W ultrasonic homogenizer (US-150T manufactured by Nissei Corp.). Thus, an aqueous composition 1 was obtained.

[Oil phase components 2]

| | |
|---|---|
| Fucoxanthin paste (fucoxanthin concentration: 15%) | 3.54 g |
| Glycerin tri(caprylate/caproate) | 4.15 g |
| Mixed tocopherol | 0.60 g |
| Ferulic acid | 0.89 g |

[Aqueous phase components 2]

| | |
|---|---|
| Sucrose lauric acid ester | 11.84 g |
| Lecithin | 1.93 g |
| Trehalose | 10.86 g |
| Ascorbic acid | 7.97 g |
| Sodium ascorbate | 7.97 g |
| Sodium carbonate (added to emulsion liquid) | 3.36 g |
| Water | 246.90 g |

Meanwhile, FUCOXANTHIN 15 (manufactured by Beijing Ginko Group, fucoxanthin 15% paste) was used as the fucoxanthin paste; COCONARD MT (HLB = 1, manufactured by Kao Corp.) was used as glycerin tri(caprylate/ caproate); RIKEN E OIL 800 (manufactured by Riken Vitamin Co., Ltd.) was used as mixed tocopherol; a ferulic acid product manufactured by Tsuno Co., Ltd. was used as ferulic acid; RYOTO SUGAR ESTER L-1695 (HLB = 16, manufactured by Mitsubishi Kagaku Foods Corp.) was used as sucrose lauric acid ester; LECION P (manufactured by Riken Vitamin Co., Ltd.) was used as lecithin; a trehalose product manufactured by Hayashibara Co., Ltd. was used as trehalose; VISCORIN (STDM) manufactured by Daiichi Fine Chemical Co., Ltd. was used as ascorbic acid; sodium L-ascorbate manufactured by Daiichi Fine Chemical Co., Ltd. was used as sodium ascorbate; and a sodium carbonate product manufactured by Kanto Chemical Co., Inc. (food additive) was used as sodium carbonate.

<Preparation of emulsion>

The oil phase composition 2 was kept warm at 70°C while stirred, and the entire amount of the oil phase composition 2 was added to the aqueous phase composition 1 that had been prepared as described above and kept warm at 70°C. The mixture was dispersed for 3 minutes with a 600-W ultrasonic homogenizer, and thus a coarsely dispersed emulsion 2 was obtained.
Subsequently, the coarsely dispersed emulsion 2 was subjected to a high pressure  emulsification treatment at a pressure of 245 MPa and at 30°C repeatedly for 4 times, using STARBURST MINI (manufactured by Sugino Machine, Ltd.), and thus an emulsion liquid was obtained.
Furthermore, sodium carbonate was added thereto under stirring to be dissolved therein, and thus an emulsion 2, which was a carotenoid-containing oil phase composition, was obtained (carotenoid concentration 0.177%).

Subsequently, the emulsion 2 thus obtained was spray dried using spray drying (Spray Dryer ADL310 type, manufactured by Yamato Scientific Co., Ltd.) under the conditions of a spray pressure of 0.15 MPa, an exit temperature of 80°C, and a throughput of 7 ml/min, and then a powder was collected with a cyclone. Thus, a powdered composition 2, which was a carotenoid-containing oil phase composition with a carotenoid concentration of 1.00%, was obtained.

[Example 3]

An oil phase composition 3 and an aqueous phase composition 3 were obtained in the same manner as in Example 1, except that the kinds and contents of the oil phase components and the aqueous phase components were changed as indicated in Table 1. Emulsification was carried out using the oil phase composition 3 and the aqueous phase composition 3 in the same manner as in Example 1, and thus an emulsion 3 was obtained (carotenoid concentration 0.36%).

Furthermore, spray drying was carried out, and thus a powdered composition 3 with a carotenoid concentration of 2.00% was obtained.

[Example 4]

An oil phase composition 4 and an aqueous phase composition 4 were obtained in the same manner as in Example 2, except that the kinds and contents of the oil phase components and the aqueous phase components were changed as indicated in Table 1. Emulsification was carried out using the oil phase composition 4 and the aqueous phase composition 4 in the same manner as in Example 2, and thus an emulsion 4 was obtained (carotenoid concentration 0.18%).

Furthermore, spray drying was carried out, and thus a powdered composition 4 with a carotenoid concentration of 1.00% was obtained.

[Example 5]

An oil phase composition 5 and an aqueous phase composition 5 were obtained in the same manner as in Example 2, except that the kinds and contents of the oil phase components and the aqueous phase components were changed as indicated in Table 1. Emulsification was carried out using the oil phase composition 5 and the aqueous phase composition 5 in the same manner as in Example 2, and thus an emulsion 5 was obtained (carotenoid concentration 0.177%).

Furthermore, spray drying was carried out, and thus a powdered composition 5 with a carotenoid concentration of 0.85% was obtained.

[Example 6]

An oil phase composition 6 and an aqueous phase composition 6 were obtained in the same manner as in Example 1, except that the kinds and contents of the oil phase components and the aqueous phase components were changed as indicated in Table 1. Emulsification was carried out using the oil phase composition 6 and the aqueous phase composition 6 in the same manner as in Example 1, and thus an emulsion 6 was obtained (carotenoid concentration 0.36%).

Furthermore, spray drying was carried out, and thus a powdered composition 3 with a carotenoid concentration of 2.00% was obtained.

[Example 7]

An oil phase composition 3 and an aqueous phase composition 3 were obtained in the same manner as in Example 1, except that the kinds and contents of the oil phase components and the aqueous phase components were changed as indicated in Table 1. Emulsification was carried out using the oil phase composition 3 and the aqueous phase composition 3 in the same manner as in Example 1, except that instead of the addition of sodium carbonate, a solution of 1 mol sodium hydroxide was added to obtain a desired pH, and the emulsion was prepared in distilled water such that the final total mass would be 300 g. Thus, an emulsion 3 was obtained (carotenoid concentration 0.36%).

Furthermore, spray drying was carried out, and thus a powdered composition 3 with a carotenoid concentration of 2.00% was obtained.

[Comparative Example 1]

An oil phase composition C1 and an aqueous phase composition C1 were obtained in the same manner as in Example 1, except that the kinds and contents of the oil phase components and the aqueous phase components were changed as indicated in Table 1. Emulsification was carried out using the oil phase composition C1 and the aqueous phase composition C in the same manner as in Example 1, and thus an emulsion C1 was obtained (carotenoid concentration 0.0.35%).

Furthermore, spray drying was carried out, and thus a powdered composition C 1 with a carotenoid concentration of 2.00% was obtained.

[Comparative Example 2]

An oil phase composition C2 and an aqueous phase composition C2 were obtained in the same manner as in Example 2, except that the kinds and contents of the oil phase components and the aqueous phase components were changed as indicated in Table 1. Emulsification was carried out using the oil phase composition C2 and the aqueous phase composition C2 in the same manner as in Example 2, and thus an emulsion C2 was obtained (carotenoid concentration 0.18%).

Furthermore, spray drying was carried out, and thus a powdered composition C2 with a carotenoid concentration of 1.00% was obtained.

[Comparative Example 3]

An oil phase composition C3 and an aqueous phase composition C3 were obtained in the same manner as in Example 1, except that the kinds and contents of the oil phase components and the aqueous phase components were changed as indicated in Table 1. Emulsification was carried out using the oil phase composition C3 and the aqueous phase composition C3 in the same manner as in Example 1, and thus an emulsion C1 was obtained (carotenoid concentration 0.35%).

Furthermore, spray drying was carried out, and thus a powdered composition C3 with a carotenoid concentration of 2.00% was obtained.

[Comparative Example 4]

An oil phase composition C4 and an aqueous phase composition C4 were obtained in the same manner as in Example 1, except that the kinds and contents of the oil phase components and the aqueous phase components were changed as indicated in Table 1. Emulsification was carried out using the oil phase composition C4 and the aqueous phase composition C4 in the same manner as in Example 1, and thus an emulsion C4 was obtained (carotenoid concentration 0.36).

Furthermore, spray drying was carried out, and thus a powdered composition C4 with a carotenoid concentration of 2.00% was obtained.

Emulsions 3 to 7 and powdered compositions 3 to 7 obtained in Examples 3 to 7 are all the carotenoid-containing compositions of Examples. Furthermore, emulsions C1 to C4 and powdered compositions C1 to C4 obtained in Comparative Examples 1 to 4 are all carotenoid-containing compositions for comparison.

Meanwhile, in Table 1, MGS-F50V (glycerin number 1, stearic acid number 1, manufactured by Nikko Chemicals Co., Ltd., HLB = 3.5) was used as glyceryl monostearate; a γ-oryzanol product manufactured by Oryza Oil & Fat Chemical Co., Ltd. was used as γ-oryzanol; DECAGLYN-50SV (manufactured by Nikko Chemicals Co., Ltd., HLB = 15) was used as decaglyceryl monostearate; FUJI FF (manufactured by Fuji Nihon Seito Corp.) was used as inulin; Calcium Ascorbate manufactured by DSM Nutriional Rroducts, Inc. was used as calcium ascorbate; a sodium hydrogen carbonate product manufactured by Kanto Chemical Co., Inc. (food additive) was used as sodium hydrogen carbonate; and a solution of 1 mol sodium hydroxide manufactured by Wako Pure Chemical Industries, Ltd. was used as sodium hydroxide.

[Table 1]

| Phase | Component | Example 1 | Example 2 | Example 3 | Example 4 | Example 5 | Example 6 | Example 7 | Comparative Example 1 | Comparative Example 2 | Comparative Example 3 | Comparative Example 4 |
|---|---|---|---|---|---|---|---|---|---|---|---|---|
| Oil phase | Lycopene paste (g) | 5.90 | | 5.90 | | | 5.90 | 5.90 | 5.90 | | 5.90 | 5.90 |
| | Fucoxanthin paste (g) | | 3.54 | | 3.54 | 2.94 | | | | 3.54 | | |
| | Diglyceryl monostearate (g) | 0.59 | | | | | 0.59 | 0.59 | 0.59 | | | |
| | Glyceryl monostearate (g) | | | 0.59 | | | | | | | 0.59 | 0.59 |
| | Glycerin tri (caprylate/caproate) (g) | | 4.15 | | 5.64 | 4.68 | | | | 4.15 | | |
| | 50% calcium ascorbate solution (g) | 4.72 | | 4.72 | | | 4.72 | 4.72 | 4.72 | | | 4.72 |
| | Ferulic acid | 1.78 | 0.89 | 1.78 | | | 1.78 | 1.78 | 1.78 | | | |
| | γ-Oryzanol | | | | | | | | | | 4.14 | 1.78 |
| | Mixed tocopherol (g) | | 0.60 | | | | | | | 0.60 | | |
| Aqueous phase | Pure water (g) | 246.90 | 246.90 | 246.90 | 246.90 | 246.90 | 246.90 | 200.00 | 246.90 | 246.90 | 246.90 | 246.90 |
| | Sucrose lauric acid ester (g) | 11.10 | 11.84 | 11.10 | 11.84 | 9.83 | 11.10 | 11.10 | 11.10 | 11.84 | | 11.10 |
| | Lecithin (g) | 1.81 | 1.93 | 1.81 | 1.93 | 1.60 | 1.81 | 1.81 | 1.81 | 1.93 | 1.81 | 1.81 |
| | Decaglyceryl monostearate (g) | | | | | | | | | | 11.10 | |
| | Trehalose (g) | 6.55 | 10.86 | | 20.42 | 7.60 | 10.09 | 6.55 | 13.62 | 30.40 | 13.62 | 8.55 |
| | Inulin (g) | | | 10.08 | | | | | | | 15.94 | 10.08 |
| | Ascorbic acid (g) | 7.97 | 7.97 | 7.97 | 2.34 | 11.58 | 7.97 | 7.97 | 7.97 | | | 4.22 |
| | Sodium ascorbate (g) | 7.97 | 7.97 | 7.97 | | | 7.97 | 7.97 | 7.97 | | | 4.22 |
| | Calcium ascorbate (g) | | | | 2.34 | 11.58 | | | | | | |
| | Sodium carbonate (g) | 7.07 | 3.60 | | 2.65 | 2.20 | 3.54 | | | | | |
| | Sodium hydrogen carbonate (g) | | | 3.54 | | | | | | | | |
| | Sodium hydroxide (1 mol solution) (g) | | | | | | | Adjusted amount | | | | |
| | Preparation water (pure water) | | | | | | | Adjusted amount | | | | 2.48 |
| Emulsion | Carotenoid concentration (%) | 0.36% | 0.18% | 0.36% | 0.18% | 0.177% | 0.36% | 0.36% | 0.35% | 0.18% | 0.35% | 0.36% |
| | pH | 9.0 | 7.5 | 7.0 | 8.0 | 8.0 | 6.7 | 9.0 | 4.3 | 6.4 | 6.3 | 7.2 |
| Powder | Carotenoid concentration (%) | 2.00% | 1.00% | 2.00% | 1.00% | 0.85% | 2.00% | 2.0% | 2.0% | 1.00% | 2.0% | 2.0% |
| | pH (after dissolution to a concentration equal to the carotenoid concentration of emulsion) | 8.5 | 7.5 | 7.2 | 7.8 | 7.8 | 6.6 | 8.8 | 4.5 | 6.6 | 6.3 | 7.3 |
| | Ascorbic acid/carotenoid (times molar amount) | 49.3 | 106.1 | 49.3 | 31.4 | 186.9 | 49.3 | 49.3 | 49.3 | 0.0 | 0.0 | 29.0 |

<Evaluation>

[1] Evaluation of properties of powdered composition and emulsion

Evaluations of the emulsions before the drying step and the powdered compositions thus obtained were carried out as follows.

(a) Evaluation of storage stability

The storage stability of the carotenoid-containing compositions was evaluated by calculating the carotenoid residual ratio (%) as follows, for the powdered compositions 1 to 7 and C1 to C4 and Emulsions 1, 2, 4, 6, and C1 to C4 obtained in various Examples and Comparative Examples.

<Carotenoid residual ratio immediately after preparation>

For the powdered compositions 1 to 7 and C1 to C4 obtained in various Examples and Comparative Examples, pure water was added to the powdered compositions to obtain a carotenoid concentration of 0.005% by volume, and thereby, emulsions were obtained. The emulsions were diluted 5.65 times and were sufficiently dissolved, and the dilutions were diluted 1062 times with acetone and were sufficiently dissolved.

Furthermore, for the emulsions 1, 2, 4, 6 and C1 to C4 obtained in various Examples and Comparative Examples, the emulsions were diluted 1062 times with acetone and were sufficiently dissolved, so as to obtain a carotenoid concentration of 0.005% by volume.

Subsequently, each of the solutions thus obtained was filtered through a 0.45-$\mu$m filter, and then for the filtrate, the absorbance at the maximum peak wavelength (465 nm to 475 nm) was measured with a spectrophotometer, V-630 (manufactured by JASCO Group).

In regard to the evaluation of the carotenoid residual ratio, in Examples 1, 3, 6 and 7, and Comparative Examples 1, 3 and 4, LYCOPENE 18 was diluted with acetone to a lycopene concentration of 0.005% by volume, similarly the absorbance at the peak wavelength was measured, and the ratio obtained by taking this absorbance of lycopene as 100% was designated as the carotenoid residual ratio for each composition.

Furthermore, in Examples 2, 4 and 5, and Comparative Example 2, FUCOXANTHIN 15 was diluted with acetone to a fucoxanthin concentration of 0.005% by volume, similarly the absorbance at the peak wavelength was measured, and the ratio obtained by taking this absorbance of fucoxanthin as 100% was designated as the carotenoid residual ratio for each composition. The results are presented in Table 2.

<Carotenoid residual ratio after passage of time>

Each of the powdered compositions 1 to 7 and C1 to C4, and emulsions 1, 2, 4, 6,  and C1 to C4 obtained in various Examples and Comparative Examples was stored for 4 months at 40°C, and then the absorbance was measured in the same manner as in the case immediately after preparation. Thus, the carotenoid residual ratio for each composition was obtained. The results are presented in Table 2.

(b) Average particle size

For each of the emulsions 1 to 7 and C1 to C4 obtained in various Examples and Comparative Examples, pure water was added to each emulsion to dilute the emulsion 20 times. For the powdered compositions 1 to 7 and C1 to C4, pure water was added to each powdered composition to a solid concentration of 1% so as to obtain an emulsion. Subsequently, the value of d = 50 at 25°C was read as the average particle size, using a particle size analyzer, FPARE-1000 (Otsuka Electronics Co., Ltd.). The results are presented in Table 2.

[Table 2]

| | | | Example 1 | Example 2 | Example 3 | Example 4 | Example 5 | Example 6 | Example 7 | Comparative Example 1 | Comparative Example 2 | Comparative Example 3 | Comparative Example 4 |
|---|---|---|---|---|---|---|---|---|---|---|---|---|---|
| Powder | Storage stability | Carotenoid residual ratio (immediately after preparation) | 100.0% | 99.8% | 99.8% | 99.4% | 100.0% | 100.0% | 99.9% | 99.7% | 99.0% | 68.5% | 99.7% |
| | | Carotenoid residual ratio (after 4 months at 40°C) | 98.0% | 91.9% | 94.3% | 81.8% | 99.4% | 82.8% | 90.2% | 46.2% | 48.7% | 33.3% | 75.3% |
| | Particle size d = 50 (nm) | | 136 | 117 | 141 | 128 | 107 | 120.0 | 137 | 118 | 119 | 350 | 137 |
| Emulsion | Storage stability | Carotenoid residual ratio (immediately after preparation) | 100.0% | 100.0% | - | 99.9% | - | 99.8% | - | 100.0% | 100.0% | 68.0% | 100.0% |
| | | Carotenoid residual ratio (after 4 months at 40°C) | 99.6% | 98.3% | - | 96.8% | - | 95.9% | - | 75.3% | 55.6% | 53.8% | 64.7% |
| | Particle size d = 50 (nm) | | 120 | 75 | 128 | 76 | 81 | 77.0 | 118 | 88 | 81 | 336 | 123 |

As can be seen from Table 1 and Table 2, it is understood that the carotenoid-containing compositions of Examples 1 to 7 have excellent storage stability, irrespective of being in an emulsion form or being in a powder form.
The entirety subject matter in the specification of Japanese Patent Application No. 2011-074846 filed on March 30, 2011 is incorporated herein by reference.

**Claims**

1. A carotenoid-containing composition comprising:

   at least one carotenoid;
   at least one selected from the group consisting of ascorbic acid, derivatives thereof and salts of the acid and the derivatives; and
   an emulsifier,
   the at least one selected from the group consisting of ascorbic acid, derivatives thereof and salts of the acid and the derivatives being contained in an amount, in terms of a number of moles of ascorbic acid, in a range of from 30 times to 190 times a total number of moles of the at least one carotenoid, and the composition having a pH in the range of from 6.5 to 9.0.

2. The carotenoid-containing composition according to claim 1, wherein the at least one carotenoid are at least one selected from lycopene or fucoxanthin.

3. The carotenoid-containing composition according to claim 1 or 2, wherein the at least one selected from ascorbic acid, derivatives thereof and salts of the acid and the derivatives, is at least one selected from L-ascorbic acid, sodium L-ascorbate, or calcium L-ascorbate.

4. The carotenoid-containing composition according to any one of claims 1 to 3, further comprising at least one selected from the group consisting of tocopherols and aromatic carboxylic acids, cinnamic acids and ellagic acids as phenolic antioxidizing agents.

5. The carotenoid-containing composition according to any one of claims 1 to 4, further comprising a water-soluble entrapping agent.

6. The carotenoid-containing composition according to any one of claims 1 to 5, further comprising a (poly)glycerin fatty acid ester having 1 to 6 glycerin units, 1 to 6 fatty acid units, and at least one hydroxyl group of a glycerin unit.

7. The carotenoid-containing composition according to any one of claims 1 to 6, wherein the composition is an oil-in-water emulsion composition containing dispersed particles which have an average particle size in a range of from 50 nm to 300 nm and contain the at least one carotenoid.

8. The carotenoid-containing composition according to claim 7, wherein the dispersed particles in the oil-in-water emulsion composition further comprise the at least one selected from the group consisting of ascorbic acid, derivatives thereof and salts of the acid and the derivatives.

9. The carotenoid-containing composition according to any one of claims 1 to 6, which is a powdered composition.

10. A method for preparing the carotenoid-containing composition according to any one of claims 1 to 7, the method comprising:

    mixing an oil phase composition containing at least one carotenoid with an aqueous phase composition containing an emulsifier and at least one selected from the group consisting of ascorbic acid, derivatives thereof and salts of the acid and the derivatives; and
    emulsifying the mixture under pressure, to obtain an oil-in-water emulsion composition.

11. The method according to claim 10, wherein the oil phase composition is obtained by mixing the at least one carotenoid with a (poly)glycerin fatty acid ester which has 1 to 6 glycerin units, 1 to 6 fatty acid units, and at least one hydroxyl group of a glycerin unit, to obtain an oil phase component mixed liquid; and then heating the oil phase component mixed liquid under temperature conditions of higher than or equal to the melting point of the at least one carotenoid.

**12.** The method for preparing a carotenoid-containing composition according to claim 10 or 11, wherein the oil phase composition contains at least one selected from the group consisting of ascorbic acid, derivatives thereof and salts of the acid and the derivatives.

**13.** The method for preparing a carotenoid-containing composition according to any one of claims 10 to 12, the method comprising drying the oil-in-water emulsion composition to obtain a powdered composition.

## INTERNATIONAL SEARCH REPORT

| | International application No. |
|---|---|
| | PCT/JP2012/057633 |

**A. CLASSIFICATION OF SUBJECT MATTER**
See extra sheet.

According to International Patent Classification (IPC) or to both national classification and IPC

**B. FIELDS SEARCHED**

Minimum documentation searched (classification system followed by classification symbols)
A61K8/31, A23L1/30, A23L1/302, A23L1/303, A61K8/55, A61K8/60, A61K8/67, A61K9/107, A61K47/10, A61K47/12, A61K47/14, A61Q5/00, A61Q11/00, A61Q13/00, A61Q15/00, A61Q19/00

Documentation searched other than minimum documentation to the extent that such documents are included in the fields searched
Jitsuyo Shinan Koho 1922-1996 Jitsuyo Shinan Toroku Koho 1996-2012
Kokai Jitsuyo Shinan Koho 1971-2012 Toroku Jitsuyo Shinan Koho 1994-2012

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 9-328419 A (BASF AG.), 22 December 1997 (22.12.1997), | 1-4,7,8,10, 12 |
| Y | claims; paragraphs [0021], [0031], [0043]; example 2 & US 5925684 A & EP 800824 A1 | 5,6,9,11,13 |
| Y | JP 2009-185023 A (Fujifilm Corp.), 20 August 2009 (20.08.2009), claims; paragraph [0040] (Family: none) | 5,9,13 |
| Y | JP 2002-316924 A (Kuraray Co., Ltd.), 31 October 2002 (31.10.2002), claims; paragraph [0008] (Family: none) | 6,11 |

☒ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 June, 2012 (07.06.12) | 19 June, 2012 (19.06.12) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
| --- |
| PCT/JP2012/057633 |

C (Continuation). DOCUMENTS CONSIDERED TO BE RELEVANT

| Category* | Citation of document, with indication, where appropriate, of the relevant passages | Relevant to claim No. |
| --- | --- | --- |
| A | JP 37-8532 B1 (Takeda Chemical Industries, Ltd.),<br>18 July 1962 (18.07.1962),<br>entire text<br>(Family: none) | 1-13 |
| A | JP 8-67666 A (Lion Corp.),<br>12 March 1996 (12.03.1996),<br>entire text<br>(Family: none) | 1-13 |

**EP 2 692 331 A1**

| INTERNATIONAL SEARCH REPORT | International application No. |
|---|---|
| | PCT/JP2012/057633 |

Continuation of A. CLASSIFICATION OF SUBJECT MATTER
  (International Patent Classification (IPC))

A61K8/31(2006.01)i, A23L1/30(2006.01)i, A23L1/302(2006.01)i,
A23L1/303(2006.01)i, A61K8/55(2006.01)i, A61K8/60(2006.01)i,
A61K8/67(2006.01)i, A61K9/107(2006.01)i, A61K47/10(2006.01)i,
A61K47/12(2006.01)i, A61K47/14(2006.01)i, A61Q5/00(2006.01)i,
A61Q11/00(2006.01)i, A61Q13/00(2006.01)i, A61Q15/00(2006.01)i,
A61Q19/00(2006.01)i

          (According to International Patent Classification (IPC) or to both national
          classification and IPC)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9157159 A
- JP 3846054 B
- JP 2006502127 A
- JP 2011074846 A

**Non-patent literature cited in the description**

- Handbook of Hydrocolloids. CRC Press, 2000, 397-403